# EUROPEAN PATENT APPLICATION

(11) **EP 4 700 114 A1**
(43) Date of publication of application: **25.02.2026**
(21) Application number: 25770215.9
(22) Date of filing: 13.05.2025
(51) Int. Cl.: C12N 1/20, A61K 35/744, A61K 35/745, A61P 1/04, A61P 31/04

(54) **COMPOUND PROBIOTIC FOR PREVENTING AND TREATING COLITIS, AND USE THEREOF**

(30) Priority: 12.07.2024 CN 202410931590
(71) Applicant: Wecare Probiotics Co., Ltd., Suzhou, Jiangsu 215200 (CN)
(72) Inventor: FANG, Shuguang, Suzhou, Jiangsu 215200 (CN); QI, Yongmei, Suzhou, Jiangsu 215200 (CN); DONG, Yao, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Prinz & Partner mbB
(86) International application number: PCT/CN2025/094574
(87) International publication number: WO 2026/011940

(57) **Abstract**

Provided are a composite probiotic for preventing and treating colitis and use thereof. The composite probiotic for preventing and treating colitis is prepared from a *Pediococcus acidilactici* PA53 strain and a *Lactobacillus helveticus* LH76 strain. The two probiotics can cooperate with each other, exhibit mutual enhancement, and have synergistic effects on the prevention and treatment of colitis, specifically manifested in: relieving the body weight loss caused by colitis, reducing the disease activity index, and recovering the colon length; improving inflammatory factor imbalance and oxidative stress responses induced by colitis; and increasing the content of short-chain fatty acid in intestinal tracts. At equivalent total bacterial counts, compared with strain interventions lacking either bacterium, the compounding of the two bacteria has a significantly improved effect on the prevention and treatment of colitis.

## Description

### TECHNICAL FIELD

The present application belongs to the field of microorganisms and relates to a composite probiotic for preventing and treating colitis and use thereof.

### BACKGROUND

Colitis, particularly ulcerative colitis, is a chronic nonspecific inflammatory disease that primarily affects the colonic mucosa and submucosa. Factors such as dysimmunity, genetic predisposition, and imbalance in gut microbiota play key roles in the pathogenesis of colitis.

The treatment of colitis primarily focuses on disease management to reduce the risk of colon cancer development in patients with colitis. Probiotics, as live microorganisms that confer beneficial effects in the human body when administered, demonstrate significant potential in the treatment of colitis. Through a series of complex mechanisms, probiotics play crucial roles in treating colitis. First, probiotics can competitively inhibit pathogenic bacteria from colonizing the intestinal epithelium, thereby effectively reducing the damage of these pathogenic bacteria to the intestinal mucosa. Then, probiotics can improve gut microecology by increasing the number of beneficial bacteria while reducing the proportion of harmful bacteria, thereby alleviating dysbiosis of intestinal flora in patients with colitis. In addition, probiotics can also stimulate the intestinal mucosal immune system, thereby enhancing local immunity and improving defensive capabilities against pathogens. Finally, probiotics can also promote the proliferation and repair of intestinal mucosal cells, thereby reducing intestinal inflammatory symptoms in patients with colitis.

The use of probiotics in the prevention and treatment of colitis is still in the initial stage, and there are still few strategies. Therefore, developing more probiotics to improve colitis can provide new ideas and methods for the treatment of colitis.

### SUMMARY

The present application provides a composite probiotic for preventing and treating colitis and use thereof.

In a first aspect, the present application provides a composite probiotic for preventing and treating colitis. The composite probiotic for preventing and treating colitis is composed of a *Pediococcus acidilactici* PA53 strain with a deposit number CGMCC No. 18798 and a *Lactobacillus helveticus* LH76 strain with a deposit number CGMCC No. 18796.

The *Pediococcus acidilactici* PA53 strain is deposited by the China General Microbiological Culture Collection Center (CGMCC) on November 4, 2019, with a deposit number of CGMCC No. 18798, at No. 1 West Beichen Road, Chaoyang District, Beijing, China.

The *Lactobacillus helveticus* LH76 strain is deposited by the China General Microbiological Culture Collection Center (CGMCC) on November 4, 2019, with a deposit number of CGMCC No. 18796, at No. 1 West Beichen Road, Chaoyang District, Beijing, China.

The present application creatively develops a new probiotic compounding method and a new strategy for preventing and treating colitis, that is, compounding and combining the *Pediococcus acidilactici* PA53 strain and the *Lactobacillus helveticus* LH76 strain. It is found that the two probiotics can cooperate with each other, exhibit mutual enhancement, and have synergistic effects on the prevention and treatment of colitis, specifically manifested in: (1) relieving the body weight loss caused by colitis, reducing the disease activity index, and recovering the colon length; (2) improving inflammatory factor imbalance and oxidative stress responses induced by colitis; and (3) increasing the content of short-chain fatty acid in intestinal tracts. At equivalent total bacterial counts, compared with strain interventions lacking either bacterium, the compounding of the two bacteria has a significantly improved effect on the prevention and treatment of colitis. Furthermore, both bacteria are probiotics, and thus the resulting products have high safety and are not prone to developing resistance.

Preferably, a ratio of the viable bacteria count of the PA53 strain to the viable bacteria count of the LH76 strain is 1:5 to 5:1, for example, 1:5, 1:4, 1:3, 1:2, 1:1, 2:1, 3:1, 4:1 or 5:1. Other specific point values within the above numerical range may be selected, and the details are not repeated here.

In a second aspect, the present application provides a probiotic agent for preventing and treating colitis. The strain in the probiotic agent is the composite probiotic described in the first aspect.

Preferably, a viable bacteria content of the PA53 strain and the viable bacteria content of the LH76 strain in the probiotic agent is not less than 1×10⁹ CFU/g or 1×10⁹ CFU/mL, respectively, for example, 1×10⁹ CFU/g (CFU/mL), 1×10¹⁰ CFU/g (CFU/mL), 5×10¹⁰ CFU/g (CFU/mL), 1×10¹¹ CFU/g (CFU/mL), 3×10¹¹ CFU/g (CFU/mL), 5×10¹¹ CFU/g (CFU/mL), 1×10¹² CFU/g (CFU/mL) or 1×10¹³ CFU/g (CFU/mL). Other specific point values within the above numerical range may be selected, and the details are not repeated here.

Preferably, a dosage form of the probiotic agent includes a solution, a lyophilized powder, a capsule, a tablet or a granule. The dosage form of the probiotic agent involved in the present application is not limited. The most common dosage forms include solutions and lyophilized powders, or the probiotic agent may further be prepared into capsules, tablets or granules.

Preferably, the dosage form of the probiotic agent is the solution, which is prepared in the following method:
inoculating the PA53 strain and the LH76 strain respectively in culture media for activation and fermentation culture in sequence to obtain fermentation broths; centrifuging the fermentation broths separately, and resuspending with a solvent to obtain a PA53 bacteria suspension and an LH76 bacteria suspension; mixing the PA53 bacterial suspension and the LH76 bacterial suspension according to a ratio of the viable bacteria count to obtain the probiotic agent.

Preferably, the dosage form of the probiotic agent is the lyophilized powder, which is prepared in the following method:
inoculating the PA53 strain and the LH76 strain respectively in culture media for activation and fermentation culture in sequence to obtain fermentation broths; centrifuging the fermentation broths separately, mixing with a protective agent, and performing lyophilization to obtain a PA53 bacteria powder and an LH76 bacteria powder; mixing the PA53 bacterial powder and the LH76 bacterial powder according to a ratio of the viable bacteria count to obtain the probiotic agent.

In a third aspect, the present application provides use of the composite probiotic described in the first aspect or the probiotic agent described in the second aspect in the preparation of a drug for preventing, ameliorating or treating colitis.

Preferably, the drug further includes an adjuvant.

Preferably, the adjuvant includes any one or a combination of at least two of a filler, an adhesive, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.

Compared with the prior art, the present application has the following beneficial effects.

The present application creatively develops a new probiotic compounding method and a new strategy for preventing and treating colitis, that is, compounding and combining the *Pediococcus acidilactici* PA53 strain and the *Lactobacillus helveticus* LH76 strain. It is found that the two probiotics can cooperate with each other, exhibit mutual enhancement, and have synergistic effects on the prevention and treatment of colitis, specifically manifested in: (1) relieving the body weight loss caused by colitis, reducing the disease activity index, and recovering the colon length; (2) improving inflammatory factor imbalance and oxidative stress responses induced by colitis; and (3) increasing the content of short-chain fatty acid in intestinal tracts. At equivalent total bacterial counts, compared with strain interventions lacking either bacterium, the compounding of the two bacteria has a significantly improved effect on the prevention and treatment of colitis. Furthermore, both bacteria are probiotics, and thus the resulting products have high safety and are not prone to developing resistance.

### BRIEF DESCRIPTION OF DRAWINGS

FIG. 1 is a graph showing the body weight change of each group of mice during modeling.
FIG. 2 is a graph showing the statistical results of the colon length of each group of mice after the experiment.
FIG. 3 is a graph showing the statistical results of the interleukin-6 level in the colon tissue of each group of mice.
FIG. 4 is a graph showing the statistical results of the interleukin-10 level in the colon tissue of each group of mice.
FIG. 5 is a graph showing the statistical results of the interleukin-1β level in the colon tissue of each group of mice.
FIG. 6 is a graph showing the statistical results of the tumor necrosis factor-α level in the colon tissue of each group of mice.
FIG. 7 is a graph showing the statistical results of myeloperoxidase activity in the colon tissue of each group of mice.
FIG. 8 is a graph showing the statistical results of superoxide dismutase activity in the colon tissue of each group of mice.
FIG. 9 is a graph showing the statistical results of the malondialdehyde content in the colon tissue of each group of mice.

### DETAILED DESCRIPTION

Technical solutions of the present application are further described below through specific examples. Those skilled in the art are to understand that the examples described herein are used for a better understanding of the present application and are not to be construed as specific limitations to the present application.

The formulation of the medium involved in the following examples is as follows:
MRS medium: 10 g/L peptone, 10 g/L beef extract, 20 g/L glucose, 2 g/L sodium acetate, 5 g/L yeast powder, 2 g/L ammonium citrate dibasic, 2.6 g/L K₂PO₄·3H₂O, 0.1 g/L MgSO₄·7H₂O, 0.05 g/L MnSO₄, 1 mL/L Tween 80, and 0.5 g/L cysteine hydrochloride.

The PA53 strain involved in the following examples is classified as *Pediococcus acidilactici,* deposited on November 4, 2019, with a deposit number of CGMCC No. 18798.

The LH76 strain involved in the following examples is classified as *Lactobacillus helveticus,* deposited on November 4, 2019, with a deposit number of CGMCC No. 18796.

The bacterial suspension involved in the following examples is prepared in the following method: a required strain is inoculated in a liquid medium and cultured for 24 h at 37 °C for activation, and the activation is continuously carried out twice to obtain an activation solution; the activation solution is inoculated in a liquid medium at an inoculum volume of 5% (v/v) and cultured for 24 h at 37 °C to obtain a bacteria solution; the bacteria solution is centrifuged at 5000 rpm at 4 °C for 10 min and filtered to obtain the bacteria; and the bacteria are resuspended with a phosphate buffer solution (PBS) to obtain the bacterial suspension.

The experiment result data were statistically analyzed using ggplot2 in the R language. Compared with the control group, ### represents p < 0.001, ## represents p < 0.01, and # represents p < 0.05. Compared with the model group, *** represents p < 0.001, **represents p < 0.01, * represents p < 0.05, and NS represents no significant difference.

### Example

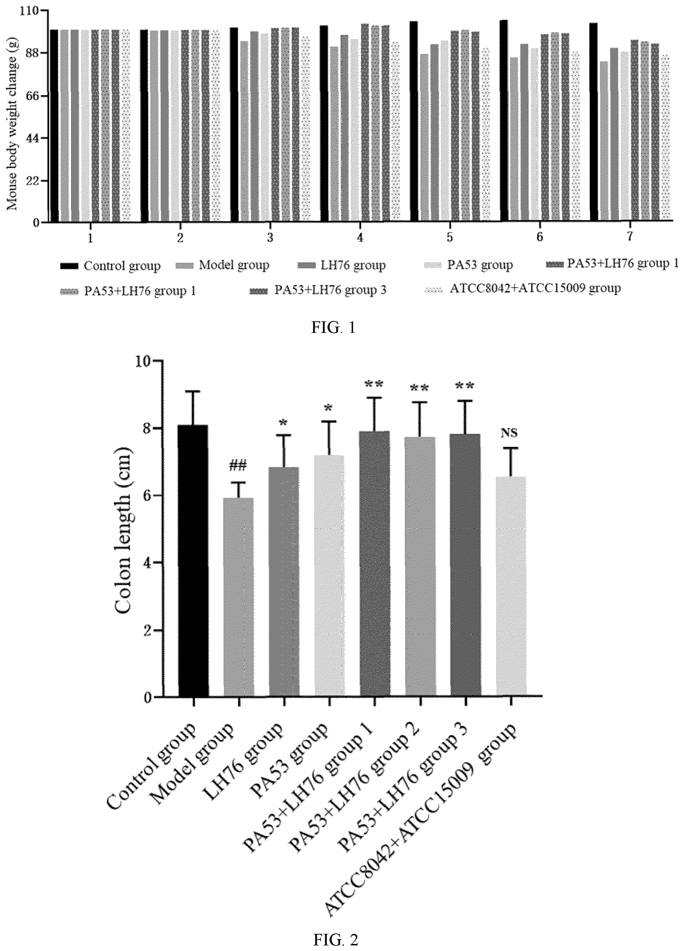

This example explores the capability of the composite probiotic to reduce symptoms in a colitis mouse model.
(1) Experimental animal: Healthy male C57BL/6 mice, 7 weeks old (64 mice), were reared in a controlled environment: with the room temperature maintained at 22 °C to 24 °C, at a humidity of 50% to 60%, and in a 12 h light/dark cycle. The mice had ad libitum access to food and water.
(2) Animal grouping: The mice were adaptively fed for one week and randomly divided into 8 groups with 8 mice in each group: a control group, a model group, a PA53 group (intervention with the PA53 bacteria solution), an LH76 group (intervention with the LH76 bacteria solution), a PA53+LH76 group 1 (compounding intervention with the PA53 bacteria solution and the LH76 bacteria solution, at a ratio of the viable bacteria count of 1:1), a PA53+LH76 group 2 (compounding intervention with the PA53 bacteria solution and the LH76 bacteria solution, at a ratio of the viable bacteria count of 5:1), a PA53+LH76 group 3 (compounding intervention with the PA53 bacteria solution and the LH76 bacteria solution, at a ratio of the viable bacteria count of 1:5), and an ATCC8042+ATCC15009 group (compounding intervention with a commercially available *Pediococcus acidilactici* ATCC8042 bacteria solution and a commercially available *Lactobacillus helveticus* ATCC15009 bacteria solution, at a ratio of the viable bacteria count of 1:1).
(3) Animal modeling and interventions:
   Throughout the entire experiment, the mice in each probiotic intervention group were fed 0.2 mL of a 12% skim milk powder solution containing probiotics (1 × 10⁹ CFU/day/mouse) daily, and the mice in the control group and the model group were fed 0.2 mL of a 12% skim milk powder solution daily. The mice in the control group had ad libitum access to water throughout the entire experiment, and the mice in the model group and each probiotic intervention group drank water freely for the first 7 days and were similarly administered drinking water containing 3.0% dextran sulfate sodium (DSS) from day 8 to day 14.
(4) Indicator analysis:
   (4.1) Body weight change of mice:
      From the first day to the seventh day of the modeling period (that is, from day 8 to day 14 of the experiment), body weights of the mice were measured daily. The measured results are shown in FIG. 1. The mice in the model group exhibited a pronounced downward trend in body weight. The probiotic interventions delayed and attenuated the weight loss of the mice to varying degrees, and especially, the compounding interventions with the PA53 strain and the LH76 strain demonstrated more significant effects on slowing down weight loss.
   (4.2) Disease activity index (DAI) change of mice:
      The disease activity index (DAI) is a critical metric for assessing colonic injury. The DAI scoring is as follows: the percentage of body weight loss, stool consistency, and fecal bleeding are comprehensively scored, and the three scores are summarized to obtain the final DAI value. For the body weight loss percentage, it is counted as 0 points for no weight change, it is counted as 1 point for 1-5% weight loss, it is counted as 2 points for 5-10% weight loss, it is counted as 3 points for 10-15% weight loss, and it is counted as 4 points for >15% weight loss; for stool consistency, it is counted as 0 points for normal stool, it is counted as 2 points for loose stool, and it is counted as 4 points for diarrhea; for fecal bleeding, it is counted as 0 points for no bleeding, it is counted as 2 points for occult blood positive, and it is counted as 4 points for gross bleeding.

The results are shown in Table 1. Compared with the control group, the DAI score of the mice in the model group was sharply increased. However, the probiotic interventions significantly reversed this increasing trend, and the compounding interventions with the PA53 strain and the LH76 strain demonstrated more pronounced reversal effects.

**Table 1**

| Group | DAI score |
|---|---|
| Control group | 0.375 |
| Model group | 9.250 |
| PA53 group | 7.250 |
| LH76 group | 7.500 |
| PA53+LH76 group 1 | 6.250 |
| PA53+LH76 group 2 | 6.125 |
| PA53+LH76 group 3 | 6.375 |
| ATCC8042+ATCC15009 group | 8.250 |

### (4.3) Colon length of mice:

After the experiment, the mice were sacrificed, and the colon length of the mice in each group was recorded. The results are shown in FIG. 2. Compared with the control group, the colon length of the mice in the model group was significantly shortened. The probiotic interventions ameliorated the colon length shortening in the mice with colitis, and the PA53+LH76 groups demonstrated the colon length restoration closest to that of the control group.

### (4.4) Changes in inflammatory markers in mice:

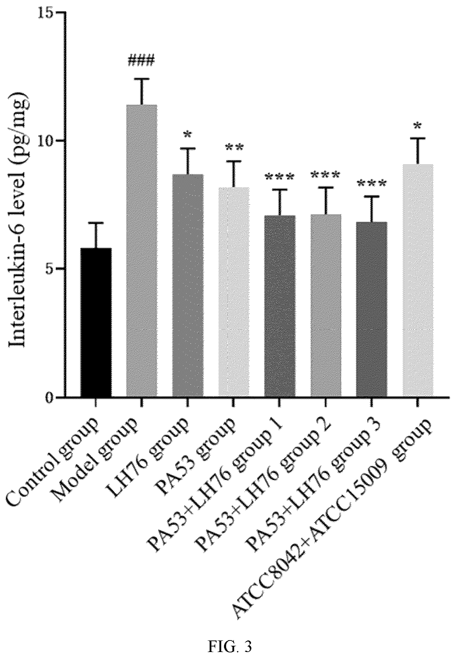

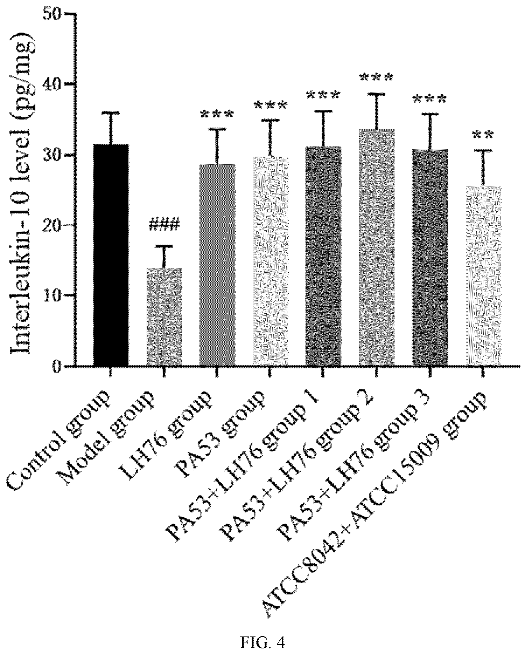

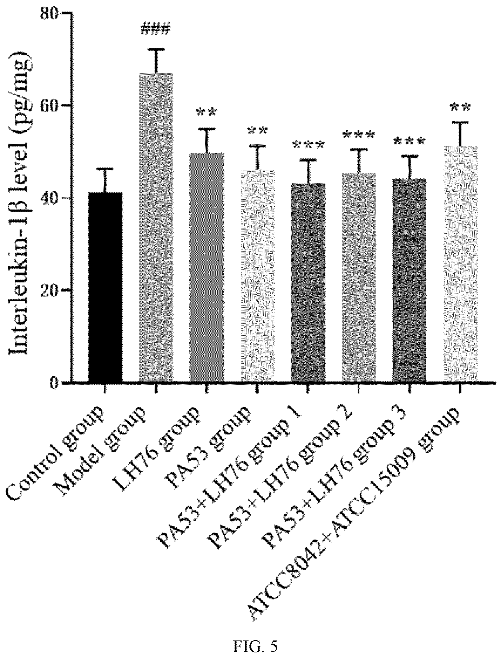

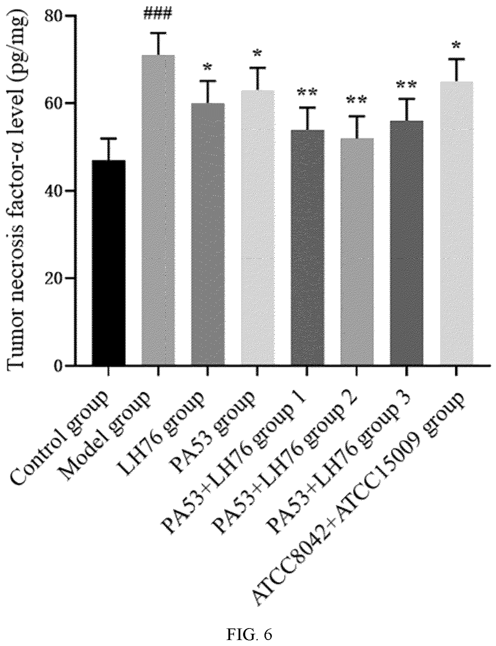

Colon tissues of the mice were homogenized and centrifuged to collect supernatants. Levels of colonic inflammatory factors were measured by using commercially available kits. The results are shown in FIGS. 3 to 6. Compared with the control group, the model group showed significantly elevated concentrations of tumor necrosis factor-α (TNF-α), interleukin-6 (IL-6), and interleukin-1β (IL-1β) in the colon of the mice and a markedly reduced interleukin-10 (IL-10) concentration in the colon tissues. The probiotic interventions significantly suppressed TNF-α, IL-6, and IL-1β concentrations and elevated the IL-10 concentration in the colon tissues. Furthermore, compared with the single-probiotic interventions, the compounding intervention with both probiotics PA53 and LH76 more effectively maintained colonic inflammatory factor balance. The above results demonstrate that the composite probiotic of the present application can promote the secretion of anti-inflammatory cytokines and suppress the secretion of pro-inflammatory cytokines, thereby alleviating colitis.

### (4.5) Changes in oxidative stress-related enzymes in mice:

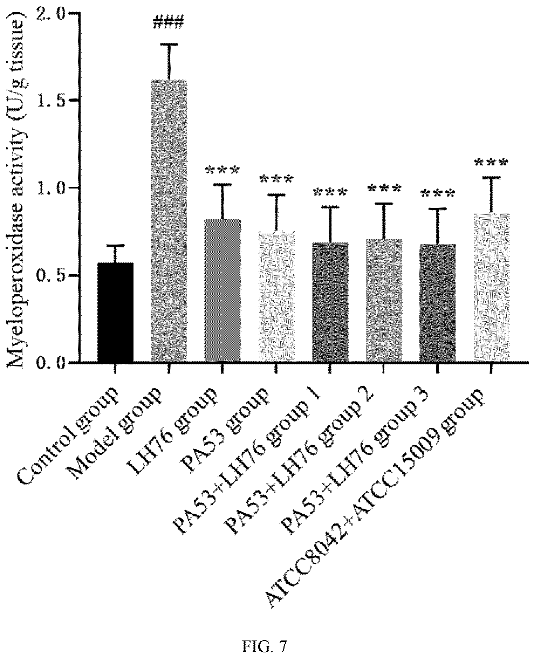

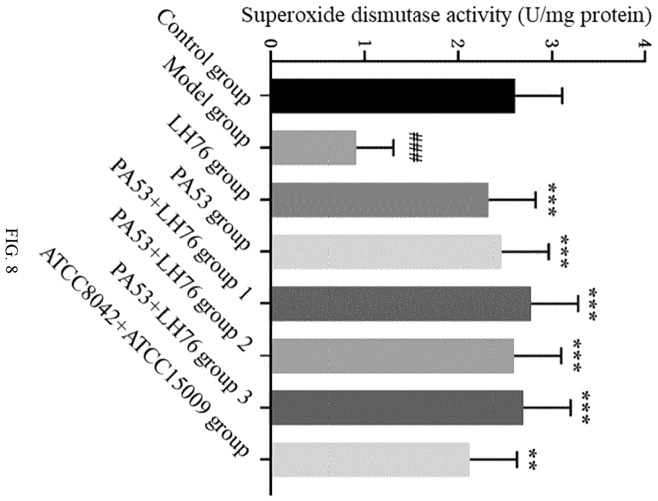

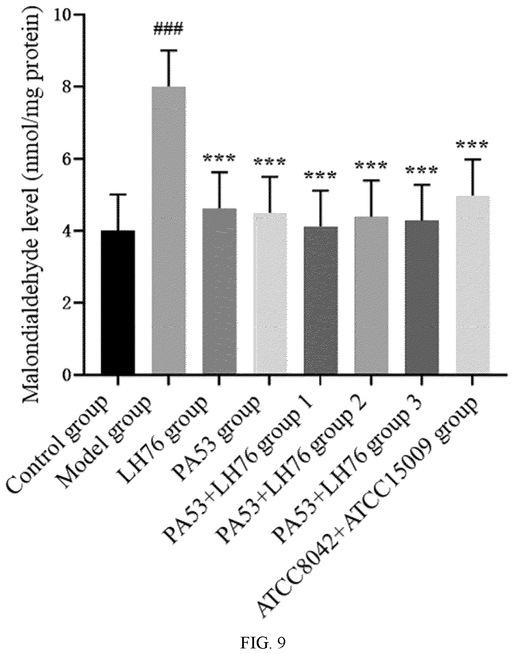

Colon tissues of the mice were homogenized and centrifuged to collect supernatants. The activities of colonic oxidative stress-related enzymes were measured by using commercially available kits. The results are shown in FIGS. 7 to 9. The myeloperoxidase (MPO) activity and the malondialdehyde (MDA) level were significantly elevated in the colon tissues of the DSS-treated mice, indicating an exacerbation of oxidative stress. However, compared with the model group, the probiotic interventions markedly reduced the MPO activity and the MDA levels in the colon tissues of the mice, indicating a significant effect of the probiotics in alleviating oxidative stress. Further analysis revealed that the superoxide dismutase (SOD) activity in the colon tissues of the model group was substantially lower than that of the control group, indicating that the antioxidant capacity of the mice in the model group was compromised in dealing with oxidative stress. The probiotic interventions significantly increased the SOD activity in DSS-induced colitis tissues, indicating that the probiotics can enhance the antioxidant capacity. Furthermore, compared with the single-probiotic interventions, the compounding intervention with both probiotics PA53 and LH76 demonstrated superior efficacy in modulating oxidative stress.

### (4.6) Changes in short-chain fatty acids:

Cecal contents of mice in each group were collected, and concentrations of short-chain fatty acids (µmol/g) were measured by gas chromatography-mass spectrometry (GC-MS). The results are shown in Table 2. Compared with the control group, the model group exhibited significantly reduced concentrations of acetic acid, propionic acid, butyric acid, and isobutyric acid. However, the probiotic interventions markedly elevated the concentrations of acetic acid, propionic acid, butyric acid, and isobutyric acid in the mice with colitis, and especially, the compounding intervention with the PA53 strain and the LH76 strain demonstrated the most pronounced concentration increase.

**Table 2**

| Short-chain fatty acid | Acetic acid | Propionic acid | Butyric acid | Isobutyric acid | Isovaleric acid |
|---|---|---|---|---|---|
| Control group | 18.82 | 6.28 | 2.18 | 2.25 | 0.39 |
| Model group | 12.78 | 4.01 | 1.19 | 1.59 | 0.23 |
| PA53 group | 16.24 | 5.60 | 1.99 | 1.88 | 0.30 |
| LH76 group | 15.56 | 5.32 | 1.92 | 1.91 | 0.29 |
| PA53+LH76 group 1 | 17.27 | 6.01 | 2.28 | 2.19 | 0.32 |
| PA53+LH76 group 2 | 17.32 | 6.11 | 2.19 | 2.02 | 0.35 |
| PA53+LH76 group 3 | 17.89 | 5.91 | 2.2 | 2.13 | 0.33 |
| ATCC8042+ATCC15009 group | 14.94 | 5.20 | 1.85 | 1.77 | 0.28 |

The applicant has stated that although the technical solutions of the present application are described through the preceding embodiments, the present application is not limited to the preceding embodiments, which means that the implementation of the present application does not necessarily depend on the preceding embodiments. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

Although the preferred embodiments of the present application have been described above in detail, the present application is not limited to the details of the above-described embodiments, and various simple modifications can be made to the technical solutions of the present application without departing from the technical concept of the present application. These simple modifications are all within the protection scope of the present application.

In addition, it is to be noted that if not in collision, specific technical features described in the preceding embodiments may be combined in any suitable manner. To avoid unnecessary repetition, various possible combination manners are not further described in the present application.

## Claims

1. A composite probiotic for preventing and treating colitis, which is composed of a *Pediococcus acidilactici* PA53 strain deposited under CGMCC No. 18798 and a *Lactobacillus helveticus* LH76 strain deposited under CGMCC No. 18796.

2. The composite probiotic for preventing and treating colitis according to claim 1, wherein a ratio of a viable bacteria count of the PA53 strain to a viable bacteria count of the LH76 strain is 1:5 to 5:1.

3. A probiotic agent for preventing and treating colitis, wherein a strain in the probiotic agent is a composite probiotic according to claim 1 or 2.

4. The probiotic agent according to claim 3, wherein a viable bacteria content of the PA53 strain and a viable bacteria content of the LH76 strain in the probiotic agent is not less than 1×10⁹ CFU/g or 1×10⁹ CFU/mL, respectively.

5. The probiotic agent according to claim 3, wherein a dosage form of the probiotic agent comprises a solution, a lyophilized powder, a capsule, a tablet or a granule.

6. The probiotic agent according to claim 5, wherein the dosage form of the probiotic agent is the solution, which is prepared in the following method:
inoculating the PA53 strain and the LH76 strain respectively in culture media for activation and fermentation culture in sequence to obtain fermentation broths; centrifuging the fermentation broths separately, and resuspending with a solvent to obtain a PA53 bacteria suspension and an LH76 bacteria suspension; mixing the PA53 bacterial suspension and the LH76 bacterial suspension according to a ratio of the viable bacteria count to obtain the probiotic agent.

7. The probiotic agent according to claim 5, wherein the dosage form of the probiotic agent is the lyophilized powder, which is prepared in the following method:
inoculating the PA53 strain and the LH76 strain respectively in culture media for activation and fermentation culture in sequence to obtain fermentation broths; centrifuging the fermentation broths separately, mixing with a protective agent, and performing lyophilization to obtain a PA53 bacteria powder and an LH76 bacteria powder; mixing the PA53 bacterial powder and the LH76 bacterial powder according to a ratio of the viable bacteria count to obtain the probiotic agent.

8. Use of the composite probiotic according to claim 1 or 2 or the probiotic agent according to any one of claims 3 to 7 in the preparation of a drug for preventing, ameliorating or treating colitis.

9. The use according to claim 8, wherein the drug further comprises an adjuvant.

10. The use according to claim 9, wherein the adjuvant comprises any one or a combination of at least two of a filler, an adhesive, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.
